# EUROPEAN PATENT APPLICATION

(11) **EP 0 916 344 A2**
(43) Date of publication of application: **19.05.1999**
(21) Application number: 98120690.7
(22) Date of filing: 06.11.1998
(51) Int. Cl.: A61K 35/36, A61K 45/06

(54) **Nef action inhibitor**

(30) Priority: 07.11.1997 JP 322096/97
(71) Applicant: NIPPON ZOKI PHARMACEUTICAL CO., LTD., Chuo-ku, Osaka (JP)
(72) Inventor: Konishi, Jin-emon, Musashino-shi, Tokyo (JP); Fuji, Yoichi, Nagoya-shi, Aichi (JP)
(74) Representative: HOFFMANN - EITLE

(57) **Abstract**

The present invention offers an inhibitor for onset of AIDS having little side effects and being capable of administering for long term and, to be more specific, to offer a drug such as an inhibitor for binding of Nef protein and an inhibitor for expression of Nef receptor.The effective component of Nef Action inhibitor of the present invention is an extract from inflammatory tissue inoculated with vaccinia virus. An extract from inflammatory tissue inoculated with vaccinia virus is highly useful as a new drug based upon a novel action mechanism of inhibition of onset of AIDS in which Nef participates. Further, it is expected that the drug exhibits far higher therapeutic effect when jointly used with virus growth inhibitors such as reverse transcriptase inhibitors and protease inhibitors.

## Description

The present invention relates to a novel pharmacological action of an extract from inflammatory tissue inoculated with vaccinia virus. More particularly, it relates to Nef action inhibitors such as an inhibitor for binding of Nef protein to Nef receptor and an inhibitor for Nef receptor expression containing the extract from inflammatory tissue inoculated with vaccinia virus as an effective component.

In general, retroviruses have structural proteins (Gag, Pol, Env) as part of a common cytoskelton. In addition to the above, human immunodeficiency virus (HIV) has regulatory proteins and accessory proteins as species-specific proteins. Nef is a kind of said accessory protein, synthesized during the initial stage of the replication cycle of the virus and is a protein specific to HIV-1, HIV-2 and SIV (simian immunodeficiency virus). When Nef was found at first, it was named as such because it was regarded as a negative factor for lowering the replicating efficiency of the virus. But, after that, as a result of studies such as infection experiments of Nef-deficient artificially modified SIV in rhesus monkey, Nef was suggested to act as a positive regulator for HIV replication and has been recognized anew as an important factor for holding a key to the pathogenesis of AIDS [Saibo Kogaku, volume 16, number 1, pages 94-99 (1997)].

As mentioned in detail in the above article by Fujii, et al, main functions of Nef in the living body are (1) promotion of virus replication, (2) CD4 down regulation and (3) cytotoxic effect on T cells. With regard to the virus infection, it has been known that Nef has an ability to bind to cell membrane, is necessary for virus adsorption and invasion of virus into cells and participates in viral DNA synthesis. Concerning the cytotoxic effect on T cells, apoptosis is induced when Nef bound to the surface of CD4⁺T cells of intestinal lymph node and peripheral blood is subjected to a cross-linking by an anti-Nef antibody and, therefore, it is strongly suggested that a binding of Nef to a Nef receptor on CD4⁺T cells greatly participates in depletion of CD4⁺T cells in HIV patients, i.e. pathogenesis of AIDS. In addition, there is a report which suggests that Nef affects the production of cytokine resulting in immunosuppression.

Most of the therapeutic agents for AIDS which have been used and developed are reverse transcriptase inhibitors such as azidothymidine, ddI, etc. and protease inhibitors, which are the drugs inhibiting the growth of virus per se. Besides them, there are immunopotentiators which increase the immunity lowered by AIDS and chemotherapeutic agents with an object of symptomatic treatment for malignant tumor and opportunistic infections accompanied by AIDS. Based upon the above-mentioned findings on Nef, a drug which inhibits the T cell apotosis induced by Nef and thereby inhibits the onset of AIDS is becoming a subject of public attention as a novel inhibitor for AIDS having an action mechanism which is different from those of the above-mentioned reverse transcriptase inhibitors and protease inhibitors.

With the known therapeutic agents for AIDS, such as the reverse transcriptase inhibitor and protease inhibitor, strong side effects and appearance of drug-resistant virus are the biggest problems. However, since Nef is a specific protein to HIV and SIV, an inhibitor for the onset of AIDS by suppresion of Nef action may have the properties that, as compared with known virus growth inhibitors, it exhibits low side effect or it is capable of standing against the change to drug-resistant virus. There has been a demand for drugs based upon such a new action mechanism, particularly for drugs having little side effect and being capable of long term administration. The present inventors have conducted various tests and studies on the pharmacological activity of an extract from inflammatory tissue inoculated with vaccinia virus and, as a result, they have found that said substance is effective in inhibiting the binding of Nef to Nef receptor and also has an action of inhibiting the expression of Nef receptor.

An object of the present invention is to offer an inhibitor for the onset of AIDS having little side effects and being capable of long term administration and, to be more specific, to offer a drug such as an inhibitor of binding of Nef protein and an inhibitor for Nef receptor expression.

It has been known that, in combating invasion from outside by viruses, etc. and progress of inner disease states, the living body produces various biofunction-regulating substances for maintaining its homeostasis and for regulating and normalizing biofunctions by means of two phases consisting of a suppressing action against excessive reactions and an enhancing action to depress functions. For example, there have been various reports on biofunction-regulating substances which are produced in inflammatory tissue inoculated with vaccinia virus, methods for extracting said substances from diseased tissues and pharmacological activities thereof (refer, for example, to the Japanese Examined Patent Publications Sho-63/039,572 B, Sho-63/025,600 B, Hei-03/043,279 B and Japanese Patent 2,594,222).

As to a drug which is actually available, there is a drug preparation of an extract from inflammatory rabbit skin inoculated with vaccinia virus. As mentioned on page 1,434 of "Drugs in Japan, Ethical Drugs" (published in August of 1994; edited by Japan Pharmaceutical Information Center; published by Yakugyo Jiho Co., Ltd.), this preparation is a drug containing a non-proteinaceous active substance extracted and isolated from inflammatory tissues of rabbits inoculated with vaccinia virus, which has been allowed for use on low back pain, neck-shoulder-arm syndromes, periarthritis scapulohumeralis, osteoarthritis, symptomatic neuralgia, itching accompanied with skin disorders (such as eczema, dermatitis and urticaria), allergic rhinitis, sequelae of subacute myelo-optico-neuropathy (such as coldness, pain and paresthesia/dysesthesia), etc., and which is approved as an ethical drug in the form of injections (subcutaneous, intramuscular and intravenous) and of tablets and is commercially available.

The effective component of inhibitors for binding of Nef protein and for Nef receptor expression of the present invention is a non-proteinaceous biofunction-regulating substance extracted from inflammatory tissues inoculated with vaccinia virus. A drug preparation of an extract from inflammatory rabbit skin inoculated with vaccinia virus which is listed in the above-mentioned "Drugs in Japan, Ethical Drugs" has been approved as a pharmaceutical agent, put onto the market and is available in Japan. In addition, various extracts from inflammatory tissue inoculated with vaccinia virus mentioned in the references such as the above-mentioned patent publications can be utilized as the active substance of the present invention and their manufacturing methods and preferred doses are illustrated in the references as well.

With regard to the route of administration to the patient, subcutaneous, intramuscular and intravenous administration by injection and oral administration by tablets are approved in the commercially available agent but it is also possible to administer pharmaceutical dosage forms other than the above-mentioned ones which are optimum for the therapy depending upon the type of the disease. The dose is to be suitably decided depending upon the kind of extract from inflammatory tissue inoculated with vaccinia virus while the dose which is approved in the commercially available preparation according to the above "Drugs in Japan, Ethical Drugs" (page 1,434) is, principally, 16 Neurotropin units per day and 3.6-7.2 Neurotropin units per day by oral administration and by injection, respectively. However, the dose may be appropriately increased or decreased depending upon the type of the disease, degree of seriousness, individual differences in the patients, method of administration, period of administration, etc.

Results of the pharmacological tests concerning the novel pharmacological action of an extract from inflammatory tissue inoculated with vaccinia virus are given below.

### Examples

### (1) Inhibiting Action for Binding of Nef to Nef Receptor on T Cell Surface

Molt-4 clone No. 8 cells (1 x 10⁶, 100 mL) were made to react with 100 mL of baculovirus gene synthesized pure Nef protein (300 mg/mL) at 4°C for one hour. The same operation was conducted in a test system to which 0.1 mL of test drug of various concentrations was added. Then the cells were washed with a phosphate buffer solution (PBS) containing 0.5% of bovine serum albumin, made to react with rabbit anti-Nef protein IgG antibody at 4°C for one hour and washed. Finally, the cells were made to react with FITC-labeled goat anti-rabbit IgG antibody at 4°C for 30 minutes followed by washing and numbers of positive cells were counted by an FACS-Caliber manufactured by Becton-Dickinson.

An example of the results is given in Table 1 and a substance of the present invention (a preparation of an extract from inflammatory skin inoculated with vaccinia virus; trade name: Neurotropin) inhibited the binding of Nef protein to T cell surface in a dose-dependent manner.

**Table 1**

| | | | | | |
|---|---|---|---|---|---|
| Concentration of Test | 0.1 | 0.05 | 0.025 | 0.0125 | 0.006 |
| Drug (NU/mL) Binding Rate (%) | 54 | 62 | 70 | 92 | 100 |

### (2) Inhibiting Action for Nef Receptor Expression on T Cell Surface

Test drug (0.1 mL) of various concentrations was added to 50 mL of Molt-4 clone No. 8 cells (1 x 10⁶) and incubated in an RPMI-1640 medium containing 10% fetal bovine serum using a 96-well microplate at 37°C for 72 hours. After washing the cells, 10 mg of anti-Nef receptor monoclonal antibody was made to react with the cells floated on 20 mL of PBS containing 0.1% of bovine serum albumin. After washing, the cells were made to react with goat anti-mouse IgM antibody labeled with FITC at 4°C for 30 minutes followed by washing and the numbers of positive cells were counted by an FACS-Caliber manufactured by Becton-Dickinson.

An example of the results is given in Table 2. The result was that the substance of the present invention inhibited the expression of Nef receptors on T cell surface in a dose-dependent manner.

**Table 2**

| | | | | | |
|---|---|---|---|---|---|
| Concentration of Test Drug (NU/mL) | 0.05 | 0.025 | 0.0125 | 0.006 | 0.003 |
| Nef Receptor Expressing Rate (%) | 82 | 85 | 93 | 99 | 101 |

### (3) Inhibiting Action for Formation of HIV-1 Symplast

Non-infected Molt-4 clone No. 8 cells (1 x 10⁵, 50 mL) which were positive for Nef receptors were incubated at 37°C for 48 hours in a 96-well microplate together with 50 mL of HIV-1 (LAV-1 strain) continuously infected Molt-4 cells (5 x 10⁴) where Nef protein was expressed on the cell surface. The numbers of the symplasts formed thereby were counted under a microscope. Test drug (0.1 mL) at various concentrations was added at the initiation of the incubation and the inhibiting action of the test drug for the symplast formation was measured. Incidentally, the ability of the test drug to form symplasts was calculated by dividing the measured value of the sample to which the test drug was added by that of the sample without test drug.

The substance of the present invention obtained by a manufacturing method mentioned in Example 1 of the Japanese Patent 2,594,222 showed a dose-dependent inhibiting action within a concentration of 0.006-0.1 mg/mL for the formation of HIV-1 symplast using the above-mentioned HIV-infected Molt-4 cells and a concentration of 0.1 mg/mL, for example, resulted in a reduction to 16%.

### (4) Inhibiting Action on Production of Virus from HIV-Infected Molt-4 Cells

Molt-4 clone No. 8 cells (1 x 10⁵, 50 mL) were infected with 50 mL of HIV-1 (LAV-1 strain) at 37°C for one hour where an M.O.I. (multiplicity of infection) was 0.02, 0.1 mL of the same test drug as above having various concentrations was added, the mixture was incubated for ten days and the amount of p24-gag antigen in a supernatant of the culture was measured whereby the amount of virus produced was determined. p24 Antigen was measured by an Abbott Antigen Measuring Kit (ELISA Kit) manufactured by Dainabot.

The result was that the substance of the present invention inhibited the production of HIV in a dose-dependent manner within a concentration of 0.006-0.1 mg/mL and a concentration of 0.1 mg/mL, for example, resulted in a reduction to 45%.

### [Merit of the Invention]

It is apparent from the results of the above-mentioned pharmacological tests that the substance of the present invention has an inhibiting action for binding of Nef protein to the T cell surface and also has an inhibiting action on expression of Nef receptors on the T cell surface. It has been suggested that Nef participates in promotion of replication of virus and in formation of symplast of infected cells as well and, since the substance of the present invention was found to have an inhibiting action to them as mentioned in the above results of the pharmacological tests, it has now been found that the substance of the present invention is highly useful as a new drug based upon a novel action mechanism of inhibition of the onset of AIDS in which Nef participates. With regard to a strategy against AIDS, various kinds of reverse transcriptase inhibitors and protease inhibitors have been developed and, moreover, a cocktail therapy where two or more of them are combined in order to fight against the mutation of HIV has been promoted. However, in addition to those inhibitors for growth of virus per se, there has been a brisk demand for drugs having a novel action mechanism and, therefore, the drug of the present invention is greatly expected as a novel drug for inhibiting the onset of AIDS. Further, it is expected that the drug which inhibits the action of Nef according to the present invention exhibits far higher therapeutic effect when jointly used with virus growth inhibitors, for example, a reverse transcriptase inhibitor such as azidothymidine (AZT), didanosine (ddI), lamivudine (3TC), sanilvudine (d4T) or zalcitabine (ddC) and a protease inhibitor such as saquinavir mesilate, nelfinavir mesilate, ritonavir or indinavir sulfate, and, accordingly, the drug of the present invention is of extremely high use.

## Claims

1. Nef action inhibitor comprising an extract from inflammatory tissue inoculated with vaccinia virus as an effective component.

2. Nef action inhibitor according to claim 1 which is an inhibitor for binding of Nef protein to Nef receptor.

3. Nef action inhibitor according to claim 1 which is an inhibitor for Nef receptor expression.

4. Nef action inhibitor according to claim 1, 2 or 3 which is used together with a reverse transcriptase inhibitor and/or a protease inhibitor.

5. Composition comprising a Nef action inhibitor according to claims 1 to 3 and a reverse transcriptase inhibitor and/or a protease inhibitor.

6. Medicament comprising a Nef action inhibitor according to claims 1 to 3 and a reverse transcriptase inhibitor and/or a protease inhibitor.

7. The use of the Nef action inhibitor according to any of claims 1 to 4 in the production of a medicament for inhibiting the action of Nef.

8. The use of the Nef action inhibitor according to any of claims 1 to 4 in the production of a medicament for inhibiting the binding of Nef protein to Nef receptor.

9. The use of the Nef action inhibitor according to any of claims 1 to 4 in the production of a medicament for inhibiting Nef receptor expression.

10. The use of the Nef action inhibitor according to any of claims 1 to 4 in the production of a medicament for the prevention or treatment of infection by a retrovirus.

11. The use according to claim 10 wherein the retrovirus is HIV.
